# EUROPÄISCHE PATENTSCHRIFT

(11) **EP 1 274 680 B1**
(45) Veröffentlichungstag und Bekanntmachung des Hinweises auf die Patenterteilung: **12.01.2005**
(21) Anmeldenummer: 01925553.8
(22) Anmeldetag: 17.04.2001
(51) Int. Cl.: C07C 401/00, C07F 7/18

(54) **SYNTHESEZWISCHENPRODUKTE ZUR HERSTELLUNG VON VITAMIN D-DERIVATEN**
SYNTHESIS INTERMEDIATE PRODUCTS FOR PRODUCING VITAMIN D DERIVATIVES
PRODUIT INTERMEDIAIRE DE SYNTHESE UTILISE EN FABRICATION DE DERIVES D DE VITAMINES

(30) Priorität: 18.04.2000 DE 10019861
(43) Veröffentlichungstag der Anmeldung: 15.01.2003
(73) Patentinhaber: Schering Aktiengesellschaft, 13353 Berlin (DE)
(72) Erfinder: STEINMEYER, Andreas, 13581 Berlin (DE); BOIDOL, Werner, 14197 Berlin (DE); ZORN, Ludwig, 13509 Berlin (DE)
(86) Internationale Anmeldenummer: PCT/EP2001/004341
(87) Internationale Veröffentlichungsnummer: WO 2001/079165

(56) Entgegenhaltungen:
- WO-A-00/53578
- HATAKEYAMA, SUSUMI ET AL: "Enantioselective synthesis of a C/D-ring synthon for the preparation of Vitamin D3 metabolites" J. CHEM. SOC., CHEM. COMMUN. (1989), (24), 1893-5 , XP002169684
- TROST, BARRY M. ET AL: "A stereocontrolled approach toward vitamin D metabolites. A synthesis of the Inhoffen-Lythgoe diol" J. AM. CHEM. SOC., Bd. 101, Nr. 15, 1979, Seiten 4378-4380, XP002169685

## Beschreibung

Vitamin D-Derivate haben in letzter Zeit erheblich an Bedeutung gewonnen, da sich neben den bekannten therapeutischen Einsatzgebieten (z.B. Vitamin D-Mangelerkrankungen, Rachitis) neue Indikationsgebiete abzeichnen. Die Entdeckung der "nichtklassischen" Vitamin D-Aktivitäten z.B. Beeinflussung des Zellwachstums von Krebszellen, Zellen der Haut oder Zellen des Immunsystems weist auf therapeutisches Potential von Vitamin D-Derivaten bei der Behandlung von Tumorerkrankungen, Hauterkrankungen sowie Störungen des Immunsystems hin.
Vitamin D-Derivate kommen in der Natur nur in äußerst geringen Mengen vor, so daß für jedwede pharmazeutische Zubereitung synthetisches Material gewonnen werden muß. Zudem lassen strukturelle Variationen des aktiven Metaboliten des Vitamin D₃, 1α,25-Dihydroxyvitamin D₃, die Herstellung von Derivaten mit veränderten Wirkprofilen zu.

Die prominenteste Synthese für Vitamin D-Derivate mit Modifikationen in der Seitenkette ist die Barton-Hesse Synthese [D.R. Andrews, D.H.R. Barton, R.H. Hesse, M.M. Pechet, Synthesis of 25-hydroxy- and 1α,25-dihydroxyvitamin D₃ from vitamin D₂ (calciferol). *J. Org.* Chem. **51:** 4819-4828 (1986), EP 0010992, EP 078704, EP 078705]. Nachteil dieser Synthese ist die Verwendung des teuren Startmaterials Vitamin D₂, welches zudem die Flexibilität der Strukturvariationen stark limitiert. So ist es zum Beispiel nicht möglich, Strukturmanipulationen am Vitamin D-Gerüst auf diesem Wege zu etablieren.

In der Literatur sind eine Reihe von partialsynthetischen Zugängen zu Vitamin D-Derivaten bekannt, die zwar einen höheres Maß an Strukturmodifikationen zulassen, aber durch Verwendung nicht allgemein zugänglicher steroidaler Vorläufer oder des Vitamin D₂ selbst keine ökonomische Rohstoffbasis darstellen.
Daneben existieren totalsynthetische Zugänge, die mit hohem Aufwand in einer Vielzahl von synthetischen Stufen die gewünschten Zielstrukturen ergeben. Eine effiziente Synthese in großem Maßstab ist aber in den beschriebenen Fällen unmöglich.

Eine konvergente Syntheseführung mit separatem Aufbau des CD-Fragmentes sowie des A-Fragmentes erscheint im Sinne einer effizienten und trotzdem variablen Synthesekonzeption günstig zu sein.
Während für die Synthese des A-Fragmentes bzw. Varianten davon bereits ausgereifte Verfahren existieren [z.B. E.G. Baggiolini et al. *J. Org. Chem.* **51**: 3098-3108 (1986), Schering AG EP 766660, WARF EP 516410], beinhaltet die Bereitstellung geeigneter CD-Fragmente noch große Probleme. Verwendet werden vielstufige Totalsynthesen mit geringer Gesamtausbeute oder Abbausequenzen hochwertiger Materialien wie z.B. Vitamin D₂. Es besteht also klar der Bedarf, CD-Fragmente aus einfacheren Vorläufern zu erzeugen und gleichzeitig eine hohes Maß an synthetischer Flexibilität zu garantieren.

Die vorliegende Erfindung betrifft die Herstellung des CD-Fragmentes [1*R*-[1α(S*),3aβ,4β,7aα]]-Octahydro-1-(2-hydroxy-1-methylethyl)-7a-methyl-5-oxo-1*H*-inden-4-propansäure I in einem mikrobiologischen Verfahren sowie das durch dieses Verfahren herstellbare Produkt.

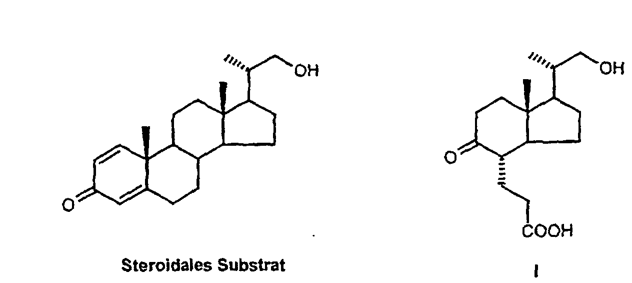

In einem Screening sind mehrere Mikroorganismen der Gattungen *Nocardioides (Arthrobacter)*und *Rhodococcus (Nocardia)* gefunden worden, die einen partiellen Abbau von Sterinen und Sterinderivaten durchführen können. Z.B. bauen die Stämme *Nocardioides simplex,* (früher *Arthrobacter simplex)* ATCC 13260 und *Rhodococcus sp.* (früher *Nocardia corallina* )ATCC 13259 die Verbindung 21-Hydroxy-20-methylpregna-1,4-dien-3-on (Δ1,4-HMP) [W.J. Marsheck et al. *Appl. Microbiol.* **23:** 72-77 (1972)] zu der CD-Verbindung I ab. Erfindungsgemäß ist der Abbau von 21-Hydroxy-20-methylpregna-1,4-dien-3-on (Δ1,4-HMP).

Werden im folgenden zwei Formeln mit z.B. **III** mit den Resten Z und R¹ oder **IIIa** mit den Resten Z² und R² bezeichnet, so unterscheiden sie sich nur dahingehend, daß in der Bedeutung von Z und R¹ gegenüber Z² und R² die Bedeutung von Wasserstoff hinzugefügt wurde, alle übrigen Bedeutungen jedoch gleich sind. Das soll für alle weiteren in der Anmeldung verwendeten Formeln analog gelten.
Die Verbindung I läßt sich in einer relativ einfachen Sequenz in ein für die Synthese von Vitamin D-Derivaten geeignetes Zwischenprodukt umwandeln. Die Erfindung betrifft daher auch ein Verfahren zur Weiterverarbeitung der Verbindung der Formel I zu einer Verbindung der Formel X und dessen Weiterverarbeitung zu einem Vitamin D Derivat.
So kann durch Veresterung der freien Carbonsäure der Carbonsäureester **IIa** generiert werden, 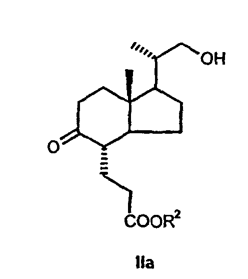 für den gilt, daß R² einen C₁-C₁₀-Alkylrest, einen C₂-C₁₀-Alkenylrest, einen C₂-C₁₀-Alkinylrest, wobei die Alkyl-, Alkenyl- und Alkinylreste substituiert sein können durch 1-5 Halogenatome, 1-5 C₁-C₅-Alkoxygruppen, und/oder unterbrochen sein können durch 1-3 Sauerstoff- oder 1-3 Schwefelatome, einen C₁-C₅-Perfluoralkylrest, einen C₁-C₅-Perchloralkylrest oder einen Benzylrest, der gegebenenfalls substituiert ist durch 1-2 Halogenatome, C₁-C₄-Alkylreste, C₁-C₄-Alkoxyreste und/oder NO₂-Gruppen bedeutet.
Prinzipiell ist jeder beliebige Ester möglich.

Bevorzugt werden Verbindungen der Formel I, worin R² ein geradkettiger oder verzweigter Alkylrest mit 1 bis 10 Kohlenstoffatomen, ein Trifluormethylrest, ein Trichlormethylrest oder ein Benzylrest, der gegebenenfalls substituiert ist durch 1-2 Halogenatome, C₁-C₄-Alkylreste, C₁-C₄-Alkoxyreste und/oder NO₂-Gruppen, bedeutet.
Der C₁-C₁₀-Alkylrest kann geradkettig oder verzweigt, und/oder substituiert sein durch 1-5 Halogenatome, 1-5 C₁-C₅-Alkoxygruppen, und/oder unterbrochen sein durch 1-3 Sauerstoff- oder Schwefelatome.
Der C₂-C₁₀-Alkenylrest kann geradkettig oder verzweigt sein, 1-3 Doppelbindungen enthalten, durch 1-5 Halogenatome, 1-5 C₁-C₅-Alkoxygruppen substituiert sein , und/oder unterbrochen sein und durch 1-3 Sauerstoff- oder 1-3 Schwefelatome. C₂-C₅-Alkenylreste mit 1 bis 2 Halogenatomen, 1-2 C₁-C₅-Alkoxygruppen, die durch ein Sauerstoffatom und/oder ein Schwefelatom unterbrochen sind, sind bevorzugt.
Der C₂-C₁₀-Alkinylrest kann geradkettig oder verzweigt sein, 1-3 Dreifachbindungen und/oder 1-3 Doppelbindungen (mindestens jedoch eine Dreifachbindung) enthalten, und/oder durch 1-5 Halogenatome, 1-5 C₁-C₅-Alkoxygruppen substituiert sein , und/oder unterbrochen sein durch 1-3 Sauerstoff- oder 1-3 Schwefelatome. C₂-C₁₀-Alkinylreste mit 1 bis 2 Halogenatomen, 1-2 C₁-C₅-Alkoxygruppen, die durch ein Sauerstoffatom und/oder ein Schwefelatom unterbrochen sind, sind bevorzugt.
Von den Substituenten des Benzylrestes werden die Methoxy- und die Nitrogruppe bevorzugt, besonders wenn diese in p-Stellung gebunden sind. Wird in der vorliegenden Anmeldung die allgemeine Formel II verwendet, so hat der Rest R¹ dieselbe Bedeutung wie R² und zusätzlich noch die Bedeutung von Wasserstoff.
Die C₁-C₅-Alkoxyreste können geradkettig oder verzweigt sein und z.B. Methoxy, Ethoxy, Propoxy, Isopropoxy bedeuten.
Halogen im erfindungsgemäßen Sinne bedeutet Fluor, Chlor, Brom oder lod.
Der C₁-C₅-Perfluoralkylrest bedeutet, CF₃, C₂F₅, C₃F₇, C₄F₉, C₅F₁₁.
Der C₁-C₅-Perchloralkylrest bedeutet, CCl₃, C₂Cl₅, C₃Cl₇, C₄Cl₉, C₅Cl₁₁·

Die freie Hydroxygruppe kann unter den bekannten Bedingungen mit einer Schutzgruppe versehen werden, wobei man die Verbindung der allgemeinen Formel **IIIa** erhält, 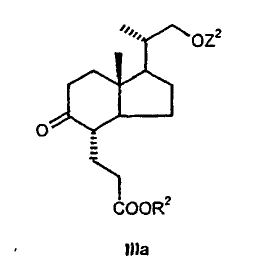 für die Z² eine alkyl-, aryl- oder gemischt alkylarylsubstituierte Silylgruppe: eine (C₁-C₄-Alkyl)₃Si-Gruppe, eine (C₆H₅)₃Si-Gruppe, eine (C₁-C₅-Alkyl)₂(C₆H₅)Si-Gruppe, eine (C₁-C₅-Alkyl) (C₆H₅)₂Si-Gruppe, wie z.B. die Gruppen Trimethylsilyl, Di-tert.butylmethylsilyl, Tert.butyldimethylsilyl, Diphenyltert.butylsilyl, Phenyldimethylsilyl, einen Tetrahydrofuranylrest, einen Tetrahydropyranylrest, eine (C₁-C₅)O(C₁-C₅)-Gruppe wie z.B. eine Methoxymethylgruppe, eine Ethoxymethylgruppe, eine Benzyleinheit, die gegebenenfalls auch substituiert sein kann durch 1-2 Halogenatome, C₁-C₄-Alkylreste und/oder C₁-C₄-Alkoxyreste, eine -CO-(C₁-C₆)-Gruppe (z.B. eine Acetylgruppe, eine Pivaloylgruppe), eine Benzoylgruppe, deren Phenylring gegebenenfalls die für den Benzylrest aufgeführten Substituenten aufweisen kann, oder eine andere gängige Schutzgruppe bedeuten kann (siehe T. W. Greene, P.G.M. Wuts *Protective Groups in Organic Synthesis,* Wiley & Sons, 1991). Die Silylgruppen: (C₁-C₄-Alkyl)₃Si-Gruppe, (C₆H₅)₃Si-Gruppe, (C₁-C₅-Alkyl)₂(C₆-H₅)Si-Gruppe, (C₁-C₅-Alkyl) (C₆H₅)₂Si-Gruppe, sowie C₁-C₅-Alkylester und (C₁-C₅)O(C₁-C₅)-Gruppen werden bevorzugt.
Wird in der Anmeldung eine Verbindung der allgemeinen Formel III aufgeführt, so hat Z der Formel III im Vergleich zu Z² der Formel IIIa dieselbe Bedeutung wie Z² mit der zusätzlichen Bedeutung Wasserstoff und R¹ der Formel III dieselbe Bedeutung wie R² der Formel IIIa mit der zusätzlichen Bedeutung Wasserstoff.
Die Ketogruppe kann nun ebenfalls mit einer Schutzgruppe versehen werden, wobei die Verbindung der allgemeinen Formel **IVa** entsteht, 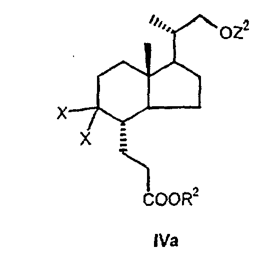 für die X,X zusammen eine geeignete Schutzgruppe für ein Keton, wie z.B. ein Ketal der Formeln -O(C₁-C5) (offenkettig), -O(C₂-C₅)O- (cyclisch), insbesondere X=OMe, OEt oder X,X= -OCH₂CH₂O-, -OCH₂CH₂CH₂O- oder ein Thioketal der Formeln -S(C₁-C₅) (offenkettig), -S(C₂-C₅)S- (cyclisch), insbesondere X=SMe, X=SEt, X=SPr, X=SBu, oder X,X= -SCH₂CH₂S-, -SCH₂CH₂CH₂S- bedeutet, die dann an beliebiger Stelle der folgenden Sequenz nach bekannten Methoden (T. W. Greene, P.G.M. Wuts *Protective Groups in Organic Synthesis,* Wiley & Sons, 1991) wieder in Freiheit gesetzt wird und gegebenenfalls zur Anknüpfung von Substituenten (z.B. durch Wittig-Reaktionen oder metallorganische Reaktionen) dient.
Alternativ kann die Ketogruppe aus den Molekül entfernt werden. Für den weiteren Syntheseverlauf ist exemplarisch dieser Fall beschrieben. Durch Umsetzung des Ketons der allgemeinen Formel **IIIa** mit Arylsulfonsäurehydraziden erhält man die Verbindung der allgemeinen Formel **Va**, 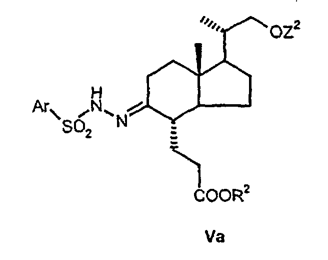 für die gilt, daß Ar eine Phenylgruppe, die 1-3fach mit einer C₁-C₅-Alkylgruppe substituiert sein kann, ist, wobei C₁-C₅-Alkyl z.B. Methyl, Ethyl, Propyl, Butyl, *t*-Butyl bedeutet. Bevorzugt wird der Tolylrest, der Xylylrest oder der 2,4,6-Trialkylphenylrest.

Durch Reduktion dieses Hydrazons **Va** mit einem Reduktionsmittel (z.B. Natriumborhydrid, Natriumtriacetoxyborhydrid, Lithiumaluminiumhydrid, Diisobutylaluminiumhydrid, Birch-Bedingungen -Li oder Na/flüssiger Ammoniak-, usw.) erhält man die Verbindung der allgemeinen Formel **VIa.**

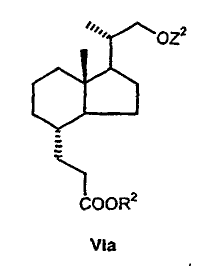

Zur Generierung eines CD-Fragmentes, welches in einem der üblichen Verfahren in ein Vitamin D-Derivat überführt werden kann, muß die Propionsäuregruppierung abgebaut werden. Zu diesem Zweck kann zunächst eine Doppelbindung in Konjugation zur Carbonsäureestereinheit eingeführt werden. Die Umsetzung des Derivates **VIa** zur Verbindung der allgemeinen Formel **VIIa** kann durch Deprotonierung mit einer Base (z.B. Lithiumdiisopropylamid, Lithiumdiethylamid, Lithiumhexamethyldisilazid, Natriumhexamethyldisilazid, Kaliumhexamethyldisilazid, Natriumhydrid, Kaliumhydrid usw.) und durch anschließenden Quench mit Phenylselenylbromid, Phenylselenylchlorid, Phenylsulfurylbromid oder Phenylsulfurylchlorid oder vergleichbaren Reagenzien sowie Oxidation und Eliminierung der schwefeloder selenhaltigen Gruppe erfolgen [z.B. H. Reich et al. *J. Am. Chem. Soc*. **95:** 5813-5817 (1972), B.M. Trost et al. *J. Am. Chem. Soc*. **98:** .4887-4902 (1972)]. Alternativ können auch andere Verfahren angewendet werden [z.B. Saegusa-Methode: I. Minami et al. *Tetrahedron* **42**: 2971-2977 (1986)].

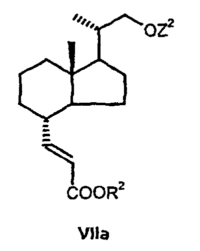

Der Abbau der ungesättigten Estereinheit kann nun durch ozonolytische Spaltung oder vergleichbare oxidative Abbauverfahren zur Verbindung der allgemeinen Formel **VIIIa** erfolgen.

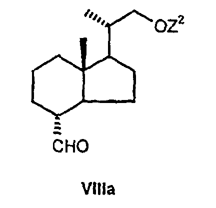

Zur optimalen Einbindung des Bausteins in bekannte Verfahren muß nun die Aldehydfunktion ihrerseits abgebaut werden. Durch Anwendung der Baeyer-Villiger-Oxidation (H₂O₂-Harnstoff-Addukt, Peressigsäure, Metachlorperbenzoesäure, Trifluorperessigsäuren usw.) oder verwandter Verfahren erhält man die Verbindung der allgemeinen Formel IXa.

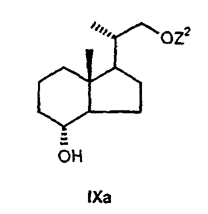

Oxidation der Hydroxygruppe mit einem Oxidationsmittel (z.B Pyridiniumchlorochromat, Pyridiniumdichromat, Swern-Bedingungen, Dess Martin-Bedingungen usw.) erhält man die Verbindung der allgemeinen Forme **Xa**

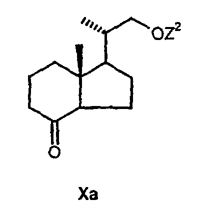

Wenn gewünscht, kann nach Spaltung der Schutzgruppe Z² nach bekannten Verfahren eine beliebige Seitenkette aufgebaut werden [z.B. E.G. Baggiolini et al. *J. Org. Chem.* **51:** 3098-3108 (1986), Schering AG: EP 421561, EP 441467 , EP 450743, EP 637299, EP 639179, EP 647219, EP 649405, EP 663902, EP 832063, EP 900198].

Ein weiterer Gegenstand der Erfindung sind die Zwischenstufen der allgemeinen Formeln III und IV, sowie die Zwischenstufen der Formeln V, VI, VII, VIII, IX und X.
Zur Fertigstellung des Vitamin D-Sytems kann das CD-Keton dann mit einem bekannten A-Ring-Fragment umgesetzt werden. Exemplarisch ist dies für die Verbindung der allgemeinen Formel **Xa** beschrieben. Umsetzung mit dem literaturbekannten A-Ring-Phosphinoxid **XI** (E.G. Baggiolini et al. s.o.) ergibt das Vitamin D-Derivat **XIIa,** 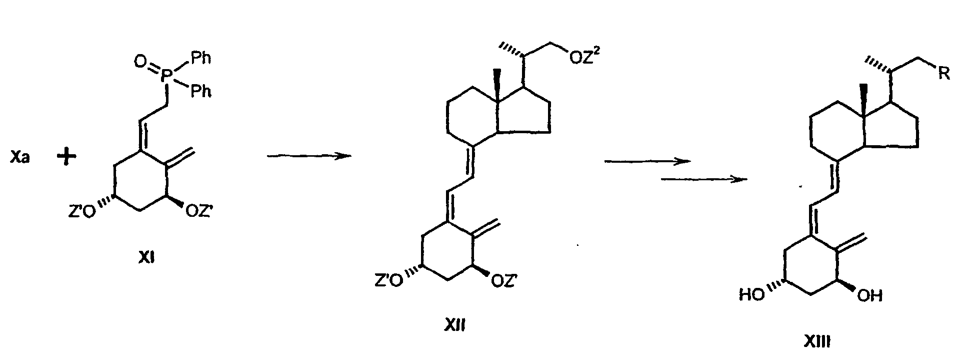 wobei für **XI** und **XII** gilt, daß Z' die insgesamt gleiche Definition wie Z² besitzen kann. Um Kompatibilität zu gewährleisten, soll aber die unabhängige Spaltung von Z² und Z' möglich sein z.B. wenn Z² = tert.Butyldimethylsilyl und Z' = tert.Butyldiphenylsilyl oder umgekehrt.
Je nach Wahl der Schutzgruppen kann jetzt entweder Z² gespalten und die Einführung der Seitenkette vorgenommen werden (siehe obige Referenzen), oder es erfolgt zunächst die Spaltung der Gruppen Z' und die Einführung von neuen Schutzgruppen Z", die unter den Spaltbedingungen für Z² stabil sind.
Darüberhinaus können auch andere bekannte A-Fragmente (z.B. **XIV, XV, XVI)** mit dem Keton **Xa** kombiniert werden [K.L. Perlman et al. *Tetrahedron Lett.* **32:** 7663-7666 (1991), S.J. Shiuey et al. J. *Org. Chem.* **55**: 243-247 (1990), R.R. Sicinski et al. *J. Med. Chem.* **41:** 4662-4674 (1998)].

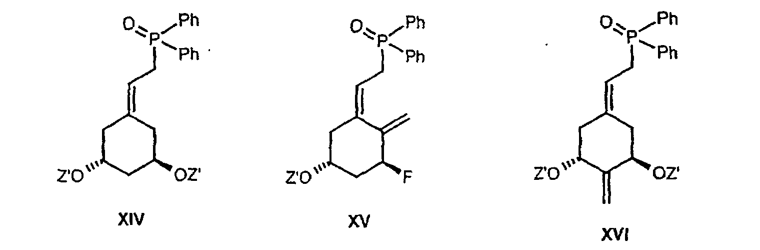

Die Verwendung des Ketons **Xa** in anderen Synthesezugängen zum Vitamin D-Gerüst ist ebenso möglich:
z.B. nach Einführung einer Bromvinyl-Einheit (**XVII**) analog B.M. Trost *J. Am. Chem. Soc.* **114:** 9836-9845 (1992) oder nach Überführung in ein Enoltriflat **(XIX)** analog L. Castedo *Tetrahedron Lett.* **29:** 1203-1206 (1988), siehe auch H.J. Knölker et al. *Tetrahedron* **53**: 91-108 (1997).

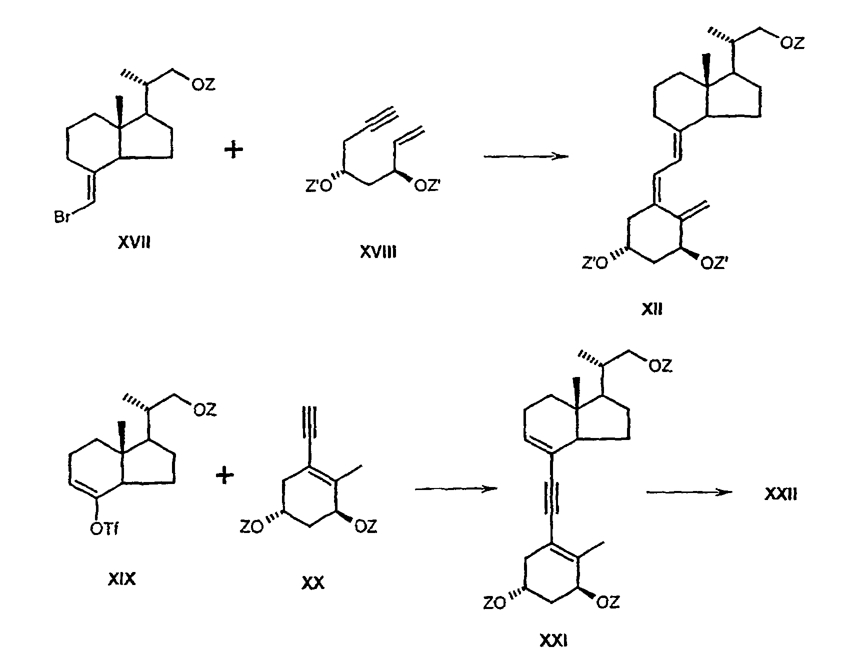

Die vorliegende Erfindung beinhaltet die direkte mikrobiologische Herstellung des CD-Fragmentes I aus einem preiswerten in großer Menge verfügbaren steroidalen Vorläufer. Durch einfache Strukturmodifikationen des CD-Fragmentes I können flexibel Vitamin D-Derivate mit Variationen an der Seitenkette, dem A-Teil und dem CD-Teil (Verwendung der Ketofunktion zur Substitution an C-9, C-11 oder C-14) erhalten werden. Das Vorliegen dreier funktioneller Gruppen am CD-Baustein **I,** in unterschiedlichen Oxidationsstufen erlaubt einfache unabhängige Manipulationen durch jedem Fachmann bekannte Schutzgruppenchemie. Der günstige Substitutionsgrad des CD-Fragements **I** ermöglicht darüberhinaus die Reduzierung der Stufenanzahl bis zur Erzeugung eines Vitamin D-Derivates gegenüber bestehenden Verfahren.
In der Literatur existieren bereits einige Prozesse zur Erzeugung von CD-Fragmenten [Henkel DE 3113053, Upjohn DE 2746323, S. Hashimoto et al. *Biochem. J.* **164,** 715-726 (1977), U. Schömer et al. *Eur. J. Appl. Mikrobiol. Biotechnol.* **10,** 99-106 (1980), T. Nakematsu et al. *Agric. Biol. Chem.* **44,** 1469-1474 (1980)]. Keine dieser Methoden erlaubt jedoch die Isolierung von CD-Fragmenten, die noch Seitenketten besitzen. Der Abbau endet in allen Fällen beim 17-Keton. Da gerade der synthetische Aufbau der Seitenkettenatome C-20 bis C-22 recht problematisch ist [P.M. Wovkulich et al. *Tetrahedron* **40,** 2283-2296 (1984)], weil ein chirales Zentrum generiert werden muß, besitzt das vorliegende Verfahren des Abbaus eines Steroids unter Erhalt des chiralen Zentrums an C-20 einen deutlichen Vorteil gegenüber den beschriebenen Methoden.

### Beispiele

Die nachfolgenden Beispiele dienen der näheren Erläuterung der Erfindungsgegenstandes, ohne ihn auf diese beschränken zu wollen.

### Hinterlegung

Die für die vorliegende Erfindung verwendeten Mikroorganismen sind bekannt und können von den folgenden Stellen bezogen werden:

### 1) Nocardioides simplex, ATCC 13260

Unter der Nummer ATCC 13260 wurde der Stamm ursprünglich als Arthrobacter simplex hinterlegt. Die aktuelle Bezeichnung lautet Nocardioides simplex. Er ist zu beziehen von der American Type Culture Collection, 10801 University Boulevard Manassas, VA 20110, Tel.:(703) 365-2700, Fax:(703) 365-2750 unter der oben genannten Nummer.

### 2) Rhodococcus sp. ATCC 13259

Unter der Nummer ATCC 13259 wurde der Stamm ursprünglich als Nocardia corallina hinterlegt. Die aktuelle Bezeichnung lautet Rhodococcus sp.. Er ist zu beziehen von der American Type Culture Collection, 10801 University Boulevard Manassas, VA 20110, Tel.:(703) 365-2700, Fax:(703) 365-2750 unter der oben genannten Nummer.

### Beispiel 1

### Herstellung der beanspruchten Verbindung I mit dem Stamm Rhodococcus sp., ATCC 13259

### Vorkultur

Ein 2 I-Erlenmeyerkolben, der 500 ml einer 30 min bei 120°C sterilisierten Nährlösung, bestehend aus 0,5% Glucose, 0,5% Hefeextrakt, 0,1% Pepton und 0,2% Corn steep liquor (pH 7,5) enthält, wird mit einer Schrägröhrchenkultur des Stammes *Rhodococcus sp.* (ATCC 13259) beimpft und 24 h bei 28°C auf einem Rotationsschüttler mit 165 upm inkubiert.

### Vorfermenter

Als Antischaummittel wurden im Vorfermenter und im Hauptfermenter Synperonic, oder bevorzugt Silicon SH der Fa. Wacker-Chemie GmbH, München, verwendet.

Mit 500 ml der Vorzucht wird ein Vorfermenter beimpft, der 10 1 steriles Medium der gleichen Zusammensetzung wie für die Vorkultur beschrieben, enthält. Die Fermentation erfolgt bei 28°C, einer Belüftungsrate von 10 l/min und einer Rührerdrehzahl von 220 upm.

### Hauptfermenter

Mit 1000 ml der 24 h alten Vorfermenter-Kultur werden 10 I steriles Medium der gleichen Zusammensetzung wie im Vorfermenter beimpft. Die Fermentation erfolgt bei 28°C und einer Belüftungsrate von 10 l/min. Die Rührerdrehzahl wird so variiert, daß der pO₂-Wert während der gesamten Fermentationsdauer größer 20% ist. Eine Zugabe des Substrats Δ1,4-HMP in mehreren Stufen, bzw. als kontinuierliche Zudosierung hat sich als vorteilhaft gegenüber einer Einmalzugabe erwiesen. Im vorliegenden Beispiel wurde das Δ1,4-HMP als Mahlung in zwei Portionen von je 10 g zugegeben. Die erste Portion wird nach einer Anwachsphase von 12 h zugegeben; nach vollständiger Umsetzung (ca. 20 h) wird die zweite Portion zugegeben. Nach weiteren 17 - 20 h ist die Reaktion beendet. Das Substrat wird bevorzugt als Mahlung eingesetzt, kann aber auch als sterilfiltrierte Lösung in z. B. Dimethylformamid zugegeben werden. Die Kontrolle der Umsetzung erfolgt unter Einsatz von Methoden wie Dünnschichtchromatographie, oder bevorzugt Gaschromatographie.

### Ernte, Aufarbeitung

Das Produkt wird aus der Kulturbrühe (9,7 1) ohne Abtrennung der Biomasse isoliert. Die Kulturbrühe wird mit 30%-iger NaOH-Lsg. auf pH 8 - 9 eingestellt und zweimal mit je 1 Vol. Methylisobutylketon extrahiert. Die wäßrige Phase wird mit 20%-iger HCl-Lsg. auf pH 2 - 3 eingestellt und dreimal mit je 1 Vol. Methylisobytylketon extrahiert. Die Extrakte werden vereinigt und am Rotationsverdampfer bis zur Trockene eingeengt. Nach Abtrennung des bei der Fermentation verwendeten Antischaummittels erhält man 17,15 g Rohprodukt I, das in Aceton gelöst und über Kieselgel filtriert wird. Nach Einengen bis zur Trockene erhält man eine weitere Rohproduktcharge I von 14,94 g. Aus diesem Rohprodukt kann das Endprodukt I durch Kristallisation aus Aceton/Diisopropylether in 67%iger Ausbeute mit einer Reinheit von >98% gewonnen werden (Fp.: 98 - 100°C).

### Weitere Umsetzungen des CD-Fragmentes I

### Beispiel 2

### [1R-[1α(S*),3aβ,4β,7aα]]-Octahydro-1-(2-hydroxy-1-methylethyl)-7a-methyl-5-oxo-1H-inden-4-propansäuremethylester 1

Man löst 10 g des CD-Fragmentes [1*R*-[1α(S*),3aβ,4β,7aα]]-Octahydro-1-(2-hydroxy-1-methylethyl)-7a-methyl-5-oxo-1*H*-inden-4-propansäure I in 300 ml Benzol, fügt 29 ml Methanol sowie eine Spatelspitze p-Toluolsulfonsäure hinzu und erhitzt die Mischung 6 h am Wasserabscheider zum Sieden. Nach dem Abkühlen neutralisiert man mit Natriumhydrogencarbonat-Lösung, extrahiert mit Essigester, wäscht die organische Phase mit Natriumchlorid-Lösung und trocknet über Natriumsulfat. Das Lösungsmittel wird im Vakuum entfernt und der Rückstand wird an Silicagel mit Essigester/Hexan chromatographiert, wobei man 9,8 g der Titelverbindung 1 als farblosen Schaum erhält.
¹H-NMR (300 MHz, CDCl₃) δ = 0,99 ppm (s, 3H); 1,03 (d, 3H); 3,38 (dd, 1H); 3,62 (dd, 1H); 3,63 (s, 3H)

### Beispiel 3

### [1R-[1α(S*),3aβ,4β,7aα]]-1-[2-[[(1,1-Dimethylethyl)diphenylsilyl]oxy]-1-methylethyl]octahydro-7a-methyl-5-oxo-1H-inden-4-propansäuremethylester 2

Man löst 5,6 g des Esters 1 in 100 ml Dimethylformamid, fügt 2,6 g Imidazol und 5,9 ml *t*-Butyldiphenylsilylchlorid hinzu und rührt die Mischung für 3 h bei Raumtemperatur. Anschließend wird Natriumchlorid-Lösung zugegeben, mit Essigester extrahiert, die organische Phase mit Natriumchlorid-Lösung gewaschen und über Natriumsulfat getrocknet. Nach dem Einengen wird der Rückstand an Silicagel mit Essigester/Hexan chromatographiert, wobei 8,6 g der Titelverbindung 2 als farbloser Schaum anfallen.
¹H-NMR (300 MHz, CDCl₃ δ = 0,95 ppm (s, 3H); 1,05 (s, 9H); 1,07 (d, 3H); 3,39 (dd, 1H); 3,60 (dd, 1H); 3,65 (s, 3H); 7,38 (m, 6H); 7,68 (4H)

### Beispiel 4

### [1R-[1α(S*),3aβ,4β,7aα]]-1-[2-[[(1,1-Dimethytethyl)diphenylsilyl]oxy]-1-methylethyl]octahydro-7a-methyl-5-[[(4-methylphenyl)sulfonyl]hydrazono]-1H-inden-4-propansäuremethylester 3

Man löst 6,3 g des Silylethers **2** in 200 ml Essigsäure und 100 ml Methanol und gibt 4,9 g p-Toluolsulfonylhydrazid hinzu. Es wird über Nacht bei Raumtemperatur gerührt und anschließend mit Natriumchlorid-Lösung gequencht. Man extrahiert mit Essigester, wäscht mit Natriumchlorid-Lösung und trocknet über Natriumsulfat. Nach dem Einengen wird der Rückstand an Silicagel mit Essigester/Hexan chromatographiert, wobei 5,2 g der Titelverbindung **3** als gelblicher Schaum anfallen.
¹H-NMR (300 MHz, CDCl₃) δ = 0,73 ppm (s, 3H); 1,04 (s, 9H); 1,05 (d, 3H); 2,42 (s, 3H); 3,38 (dd, 1H); 3,58 (dd, 1H); 3,69 (s, 3H); 7,30 (d, 2H); 7,38 (m, 6H); 7,67 (4H); 7,85 (d, 2H)

### Beispiel 5

### [1R-[1αa(S*),3aβ,4β,7aα]]-1-[2-[[(1,1-Dimethylethyl)diphenylsilyl]oxy]-1-methylethyl]octahydro-7a-methyl-1H-inden-4-propansäuremethylester 4

Man löst 5,2 g des Tosylhydrazons 3 in 150 ml Essigsäure und gibt 1,42 g Natriumborhydrid hinzu. Es wird 4 h bei Raumtemperatur gerührt und anschließend mit verdünnter Natronlauge gequencht. Man extrahiert mit Essigester, wäscht mit Natriumchlorid-Lösung und trocknet über Natriumsulfat. Das Lösungsmitttel wird entfernt und der Rückstand wird an Silicagel mit Hexan/Essigester chromatographiert, wobei man 2,7 g der Titelverbindung 4 erhält.
¹H-NMR (300 MHz, CDCl₃) δ = 0,61 ppm (s, 3H); 1,03 (s, 9H); 1,06 (d, 3H); 3,35 (dd, 1H); 3,60 (dd, 1H); 3,64 (s, 3H); 7,38 (m, 6H); 7,68 (m, 4H)

### Beispiel 6

### [1R-(1α(S*),3aβ,4β(E),7aα]]-3-[1-[2-[[(1,1-Dimethylethyl)diphenylsilyl]oxy]-1-methylethyl]octahydro-7a-methyl-1H-inden-4-yl]prop-2-ensäuremethylester 5

Man stellt 0,57 mmol Lithiumdiisopropylamid in Tetrahydrofuran her und gibt bei -78°C 100 mg des Esters **4** in 2 ml Tetrahydrofuran gefolgt von 1 ml 1,3-Dimethyltetrahydro-2(1H)-pyrimidinon (DMPH) hinzu. Nach 10 min bei dieser Temperatur werden 135 mg Phenylselenylbromid zugetropft und man rührt 90 min nach. Anschließend quencht man durch Zugabe von Ammoniumhydrochlorid-Lösung, extrahiert mit Essigester, wäscht mit Natriumchlorid-Lösung und trocknet über Natriumsulfat. Das Rohprodukt löst man in 3 ml Tetrahydrofuran, kühlt aus 0°C und setzt 0,2 ml WasserstoffperoxidLösung (30%) hinzu. Nach einer Stunde bei 0°C wird Natriumhydrogensulfat-Lösung zugegeben, mit Essigester extrahiert, die organische Phase mit Natriumthiosulfat-Lösung und Natriumchlorid-Lösung gewaschen und über Natriumsulfat getrocknet. Man entfernt das Lösungsmittel und chromatographiert den Rückstand an Silicagel mit Hexan/Esigester, wobei man 78 mg der Titelverbindung 5 als farbloser Schaum erhält.
¹H-NMR (300 MHz, CDCl₃) δ = 0,69 ppm (s, 3H); 1,03 (s, 9H); 1,08 (d, 3H); 3,39 (dd, 1H); 3,64 (dd, 1H); 3,71 (s, 3H); 5,76 (d, 1H); 6,78 (dd, 1H); 7,37 (m, 6H); 7,68 (m, 4H)

### Beispiel 7

### [1R-[1α(S*),3aβ,4β,7aα]]-1-[2-1[[(1,1-Dimethylethyl)diphenysilyl]oxy]-1-methylethyl]octahydro-7a-methyl-1H-inden-4-carbaldehyd6

Man löst 830 mg des Esters **5** in 10 ml Dichlormethan und 5 ml Methanol und leitet bei -78°C ein Ozon/Sauerstoffgemisch, welches in einem Ozongenerator erzeugt wird, hindurch, bis die Lösung eine blaue Farbe besitzt. Anschließend werden 500 mg Triphenylphosphin zugegeben und man läßt das Gemisch auf Raumtemperatur erwärmen. Das Reaktionsgemisch wird eingeengt und an Silicagel mit Essigester/Hexan chromatographiert, wobei 650 mg der Titelverbindung 6 als farbloser Schaum anfallen.
¹H-NMR (300 MHz, CDCl₃) δ = 0,69 ppm (s, 3H); 1,04 (s, 9H); 1,10 (d, 3H); 3,39 (dd, 1H); 3,61 (dd, 1H); 7,37 (m, 6H); 7,68 (m, 4H); 9,53 (d, 1H)

### Beispiel 8

### [1R-[1α(S*),3aβ,4β,7aα]]-1-[2-[[(1,1-Dimethytethyl)diphenylsilyl]oxy]-1-methytethyl]octahydro-7a-methyl-1H inden-4-ol 7

Man löst 506 mg Harnstoff-Wasserstoffperoxid-Addukt und 714 mg Kaliumdihydrogenphosphat in 8 ml Dichlormethan und tropft den Aldehyd 6 in 10 ml Dichlormethan zu. Anschließend tropft man 0,15 ml Trifluoressigsäureanhydrid so langsam hinzu, daß die Temperatur der Lösung 35°C nicht übersteigt. Nach dem Rühren über Nacht stellt man mit 4 N Natronlauge den pH-Wert auf 6 ein und tropft dann Natriumhydrogencarbonat-Lösung zu, bis die Gasentwicklung beendet ist. Es wird mit Dichlormethan extrahiert, über Natriumsulfat getrocknet und das Lösungsmittel im Vakuum entfernt. Der Rückstand wird an Silicagel mit Essigester/Hexan chromatographiert, wobei man 375 mg der Titelverbindung 7 als farblosen Schaum erhält.
¹H-NMR (300 MHz, CDCl₃) δ = 0,66 ppm (s, 3H); 1,05 (s, 9H); 1,09 (d, 3H); 3,38 (dd, 1H); 3,54 (m, 1H); 3,61 (dd, 1H); 7,37 (m, 6H); 7,68 (m, 4H)

### Beispiel 9

### [1R-[1α(S*),3aβ,7aβ]]-1-[2-[[(1,1-Dimethylethyl)diphenylsilyl]oxy]-1-methylethyl]octahydro-7a-methyl-4H-inden-4-on 8

Man löst 47 mg des Alkohols 7 in 4 ml Dichlormethan, fügt 32 mg Pyridiniumchlorochromat zu und rührt für 4 h bei Raumtemperatur. Anschließend wird Diethylether zugegeben und über Celite filtriert. Das Solvens des Filtrates wird entfernt und der Rückstand wird an Silicagel mit Essigester/Hexan chromatographiert, wobei man 39 mg der Titelverbindung 8 erhält.
¹H-NMR (300 MHz, CDCl₃) δ = 0,61 ppm (s, 3H); 1,05 (s, 9H); 1,11 (d, 3H); 3,40 (dd, 1H); 3,61 (dd, 1H); 7,37 (m, 6H); 7,68 (m, 4H)

## Patentansprüche

1. Verbindungen der allgemeinen Formel III 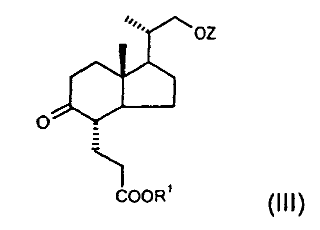 worin
R¹ ein Wasserstoffatom, einen C₁-C₁₀-Alkylrest, einen C₂-C₁₀-Alkenylrest, einen C₂-C₁₀-Alkinylrest, wobei die Alkyl-, Alkenyl- und Alkinylreste substituiert sein können durch 1-5 Halogenatome, 1-5 C₁-C₅-Alkoxygruppen, und/oder unterbrochen sein können durch 1-3 Sauerstoff- oder 1-3 Schwefelatome, einen C₁-C₅-Perfluoralkylrest, einen C₁-C₅-Perchloralkylrest oder einen Benzylrest, der gegebenenfalls substituiert ist durch 1-2 Halogenatome, C₁-C₄-Alkylreste, C₁-C₄-Alkoxyreste und/oder NO₂-Gruppen, und
Z ein Wasserstoffatom oder eine Hydroxyschutzgruppe und bedeutet.

2. Verbindungen der allgemeinen Formel IV 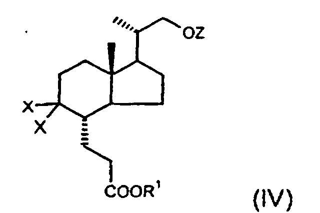 worin
R¹ ein Wasserstoffatom, einen C₁-C₁₀-Alkylrest. einen C₂-C₁₀-Alkenylrest, einen C₂-C₁₀-Alkinylrest, wobei die Alkyl-, Alkenyl- und Alkinylreste substituiert sein können durch 1-5 Halogenatome, 1-5 C₁-C₅-Alkoxygruppen, und/oder unterbrochen sein können durch 1-3 Sauerstoff- oder 1-3 Schwefelatome, einen C₁-C₅-Perfluoralkylrest, einen C₁-C₅-Perchloralkylrest oder einen Benzylrest, der gegebenenfalls substituiert ist durch 1-2 Halogenatome, C₁-C₄-Alkylreste, C₁-C₄-Alkoxyreste und/oder NO₂-Gruppen,
Z ein Wasserstoffatom oder eine Hydroxyschutzgruppe und
X,X gemeinsam ein Ketosauerstoffatom oder eine Ketoschutzgruppe bedeutet.

3. Verbindungen der allgemeinen Formel IV nach Anspruch 2 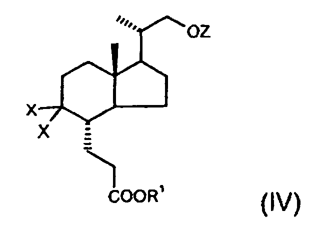 worin
R¹ ein Wasserstoffatom, einen C₁-C₁₀-Alkylrest, einen C₂-C₁₀-Alkenylrest, einen C₂-C₁₀-Alkinylrest, wobei die Alkyl-, Alkenyl- und Alkinylreste substituiert sein können durch 1-5 Halogenatome, 1-5 C₁-C₅-Alkoxygruppen, und/oder unterbrochen sein können durch 1-3 Sauerstoff- oder 1-3 Schwefelatome, einen C₁-C₅-Perfluoralkylrest, einen C₁-C₅-Perchloralkylrest oder einen Benzylrest. der gegebenenfalls substituiert ist durch 1-2 Halogenatome, C₁-C₄-Alkylreste, C₁-C₄-Alkoxyreste und/oder NO₂-Gruppen,
Z ein Wasserstoffatom, eine (C₁-C₄-Alkyl)₃Si-Gruppe, eine (C₆H₅)₃Si-Gruppe, eine (C₁-C₅-Alkyl)₂(C₆H₅)Si-Gruppe, eine (C₁-C₅-Alkyl) (C₆H₅)₂Si-Gruppe, eine Tetrahydrofuranylgruppe, eine Tetrahydropyranylgruppe, eine (C₁-C₅)O(C₁-C₅)-Gruppe, eine gegebenenfalls durch 1-2 Halogenatome, C₁-C₄-Alkylreste, C₁-C₄-Alkoxyreste oder NO₂-Gruppen substituierte Benzylgruppe, eine -CO-(C₁-C₆)-Gruppe, oder eine gegebenenfalls durch 1-2 Halogenatome, C₁-C₄-Alkylreste, C₁-C₄-Alkoxyreste und/oder NO₂-Gruppen substituierte Benzoylgruppe und
X,X eine -O(C₁-C₅)-Gruppe, eine -S(C₁-C₅)-Gruppe, gemeinsam ein Ketosauerstoffatom, eine cyclische Gruppe -O(C₂-C₅)O-, oder eine cyclische Gruppe -S(C₂-C₅)S- oder eine C₆H₅-SO₂-NH-N-Gruppe, deren Phenylrest gegebenenfalls 1-3fach substituiert ist mit einem C₁-C₅-Alkylrest, bedeutet.

4. Verbindung der Formel I, nach Anspruch 1

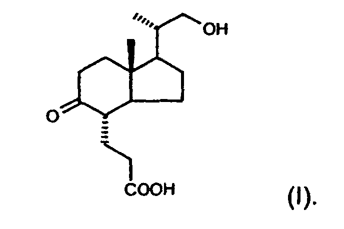

5. Verfahren zur Herstellung der Verbindung der Formel I nach Anspruch 4, **dadurch gekennzeichnet, dass** die Verbindung der Formel I durch eine Abbaureaktion der steroidalen Vorstufe der Formel 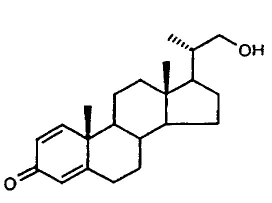 mit einem Mikroorganismus der Gattungen Nocardioides oder Rhodococcus erhalten wird.

6. Verfahren nach Anspruch 5, **dadurch gekennzeichnet, dass** einer der beiden Mikroorganismen *Nocardioides simplex,* ATCC 13260 oder *Rhodococcus sp.* ATCC 13259 ist.

7. Verfahren zur Weiterverarbeitung der Verbindung der allgemeinen Formel I 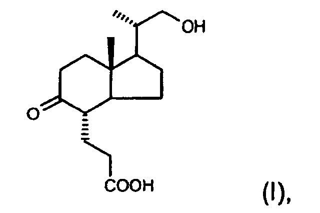 die in einem mikrobiologischen Schritt durch Abbau des steroidalen Vorläufers gemäß Anspruch 5 erhalten wurde, **dadurch gekennzeichnet, dass** deren funktionelle Gruppen, wenn gewünscht, in beliebiger Reihenfolge geschützt werden, die Ketogruppe, gegebenenfalls überführt wird in eine geeignete Vorstufe, reduziert wird zur Methylengruppe und die Propionsäurekette in einer beliebigen Reaktionsfolge abgebaut wird zur Ketofunktion und im Anschluss gegebenenfalls die Hydroxyschutzgruppe entfernt wird und/oder gegebenenfalls andere Schutzgruppen eingeführt werden und eine Verbindung der allgemeinen Formel X 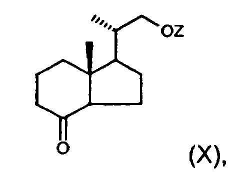 erhalten wird, wobei Z die oben angegebenen Bedeutung hat.

8. Verfahren nach Anspruch 7, **dadurch gekennzeichnet, dass** die Verbindung der Formel (X) weiterverarbeitet wird in beliebigen Reaktionsschritten zu Vitamin D oder Vitamin D Derivaten.

9. Verfahren nach Anspruch 7, **dadurch gekennzeichnet, dass** die Verbindung der Formel I in einen Ester der allgemeinen Formel **IIa** 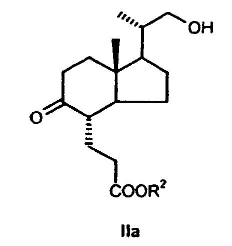 überführt wird, dessen freie Hydroxygruppe geschützt wird, um eine Verbindung der allgemeinen Formel IIIa 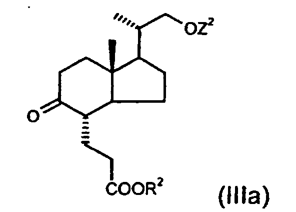 zu erhalten,
worin Z² die Bedeutung von Z hat, aber nicht Wasserstoff bedeutet und R² die Bedeutung von R¹ hat, aber nicht Wasserstoff bedeutet,
deren Ketogruppe unter üblichen Bedingungen in das Tosylhydrazon Va 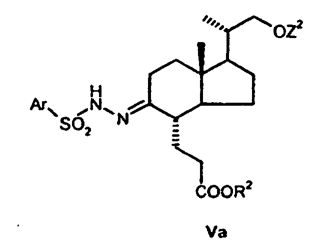 überführt wird,
worin Ar eine Phenylgruppe, die gegebenenfalls 1-3fach substituiert ist durch eine C₁-C₅-Alkylgruppe, bedeutet,
das nach Reduktion mit einem Reduktionsmittel wie Natriumborhydrid, Natriumtriacetoxyborhydrid, Lithiumaluminiumhydrid, Diisobutylaluminiumhydrid
oder unter Birch-Bedingungen
reduziert wird zu einer Verbindung der allgemeinen Formel VIa 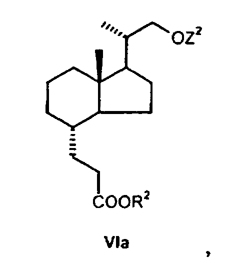 wobei, falls die Schutzgruppen unter den Bedingungen der Reduktion und deren Aufarbeitung abgespalten worden sein sollten, Schutzgruppen wieder eingeführt werden, und
deren Propionsäureseitenkette dann durch die Reaktionsfolge
a) Einführen einer 2,3-Doppelbindung mit Litiumdiisopropylamid/DMPH/Phenylselenylbromid/Wasserstoffperoxid
b) Ozonolyse
c) Hamstoff/Wasserstoffperoxid-Oxidation
d) Pyridiniumchlorochromat-Oxidation
abgebaut und ins Keton Xa 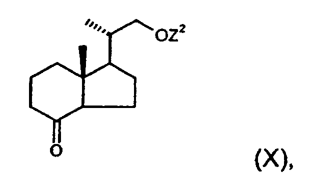 überführt wird.

10. Zwischenprodukte des Verfahrens nach Anspruch 9 der allgemeinen Formeln
Va, 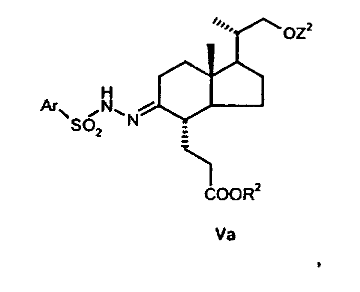 VIa, 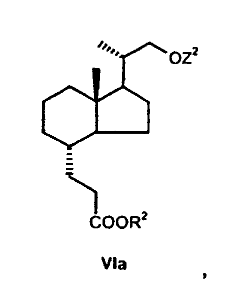 VIIa 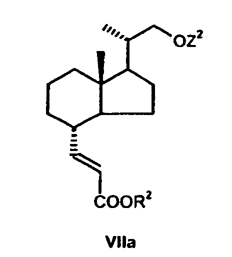 VIIIa 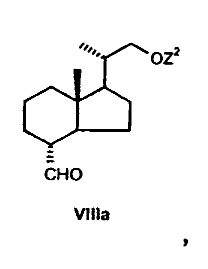 IXa 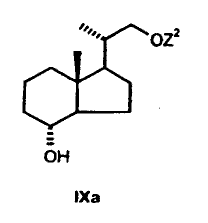 und Xa 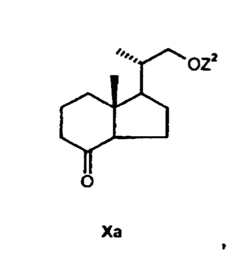 sowie deren Analoge, in denen Z² die Bedeutung von Z und R² die Bedeutung von R¹ hat, mit der Maßgabe, dass Verbindungen der Formel IXa ausgeschlossen sind, in der Z² tert. Butyldimethylsilyl oder Wasserstoff bedeutet.

11. Verwendung der Verbindungen der allgemeinen Formeln I, III, VIIa, und VIIIa zur Synthese von Vitamin D Derivaten.

## Claims

1. Compounds of general formula III 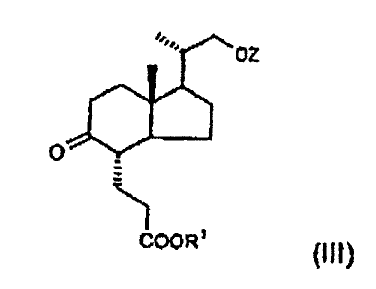 in which
R¹ means a hydrogen atom, a C₁-C₁₀-alkyl radical, a C₂-C₁₀-alkenyl radical, a C₂-C₁₀-alkinyl radical, where the alkyl, alkenyl and alkinyl radicals can be substituted by 1-5 halogen atoms, 1-5 C₁-C₅-alkoxy groups, and/or can be interrupted by 1-3 oxygen atoms or 1-3 sulphur atoms, a C₁-C₅-perfluoroalkyl radical, a C₁-C₅-perchloroalkyl radical or a benzyl radical, which optionally is substituted by 1-2 halogen atoms, C₁-C₄-alkyl radicals, C₁-C₄-alkoxy radicals and/or NO₂ groups, and
Z means a hydrogen atom or a hydroxy protective group.

2. Compounds of general formula IV 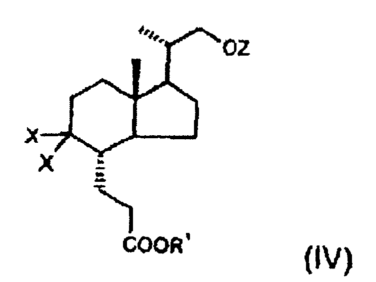 in which
R¹ means a hydrogen atom, a C₁-C₁₀-alkyl radical, a C₂-C₁₀-alkenyl radical, a C₂-C₁₀-alkinyl radical, where the alkyl, alkenyl and alkinyl radicals can be substituted by 1-5 halogen atoms, 1-5 C₁-C₅-alkoxy groups, and/or can be interrupted by 1-3 oxygen atoms or 1-3 sulphur atoms, a C₁-C₅-perfluoroalkyl radical, a C₁-C₅-perchloroalkyl radical or a benzyl radical, which optionally is substituted by 1-2 halogen atoms, C₁-C₄-alkyl radicals, C₁-C₄-alkoxy radicals and/or NO₂ groups,
Z means a hydrogen atom or a hydroxy protective group, and
X,X together means a keto-oxygen atom or a keto protective group.

3. Compounds of general formula IV according to Claim 2 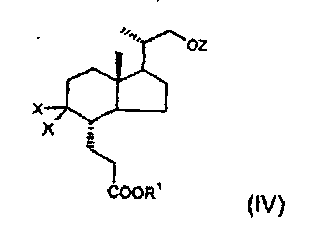 in which
R¹ means a hydrogen atom, a C₁-C₁₀-alkyl radical, a C₂-C₁₀-alkenyl radical, a C₂-C₁₀-alkinyl radical, where the alkyl, alkenyl and alkinyl radicals can be substituted by 1-5 halogen atoms, 1-5 C₁-C₅-alkoxy groups, and/or can be interrupted by 1-3 oxygen atoms or 1-3 sulphur atoms, a C₁-C₅-perfluoroalkyl radical, a C₁-C₅-perchloroalkyl radical or a benzyl radical, which optionally is substituted by 1-2 halogen atoms, C₁-C₄-alkyl radicals, C₁-C₄-alkoxy radicals and/or NO₂ groups,
Z means a hydrogen atom, a (C₁-C₉-alkyl)₃Si group, a (C₆H₅)₃Si group, a (C₁-C₅-alkyl)₂(C₆H₅)Si group, a (C₁-C₅-alkyl) (C₆H₅) ₂Si group, a tetrahydrofuranyl group, a tetrahydropyranyl group, a (C₁-C₅) O (C₁-C₅) group, a benzyl group that is optionally substituted by 1-2 halogen atoms, C₁-C₄-alkyl radicals, C₁-C₄-alkoxy radicals or NO₂ groups, a -CO-(C₁-C₆) group, or a benzoyl group that is optionally substituted by 1-2 halogen atoms, C₁-C₄-alkyl radicals, C₁-C₄-alkoxy radicals and/or NO₂ groups, and
X,X means an -O (C₁-C₅) group, an -S(C₁-C₅) group, together a keto-oxygen atom, a cyclic group -O(C₂-C₅) O-, or a cyclic group -S(C₂-C₅)S- or a C₆H₅-SO₂-NH-N group, whose phenyl radical is optionally substituted in 1-3 places with a C₁-C₅-alkyl radical.

4. Compound of formula I, according to Claim 1

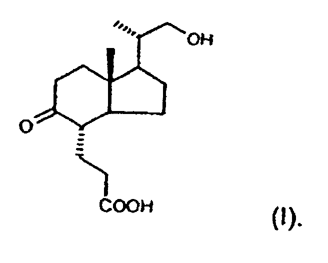

5. Process for the production of the compound of formula I according to Claim 4, **characterized in that** the compound of formula I is obtained by a degradation reaction of the steroidal precursor of formula 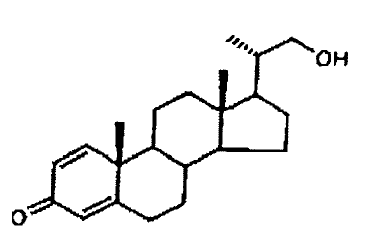 with a microorganism of the genera Nocardioides or Rhodococcus.

6. Process according to Claim 5, **characterized in that** one of the two microorganisms is *Nocardioides simplex,* ATCC 13260, or *Rhodococcus sp.* ATCC 13259.

7. Process for further processing of the compound of general formula I 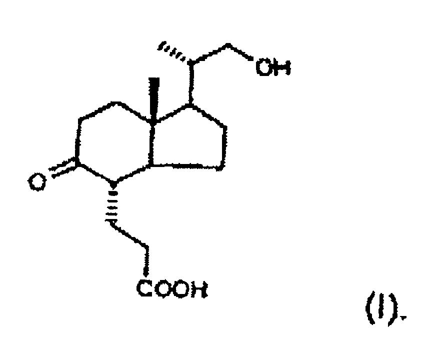 that was obtained in a microbiological step by degradation of the steroidal precursor according to Claim 5, **characterized in that** the functional groups thereof, if desired, are protected in any sequence; the keto group is optionally converted into a suitable precursor, reduced to the methylene group, and the propionic acid chain is degraded in any reaction sequence to the keto function and subsequently optionally the hydroxy protective group is removed and/or optionally other protective groups are introduced, and a compound of general formula X 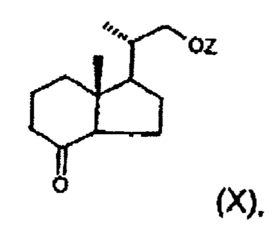 is obtained, where Z has the above-indicated meaning.

8. Process according to Claim 7, **characterized in that** the compound of formula (X) is further processed in any reaction steps to vitamin D or vitamin D derivatives.

9. Process according to Claim 7, **characterized in that** the compound of formula I is converted into an ester of general formula **IIa** 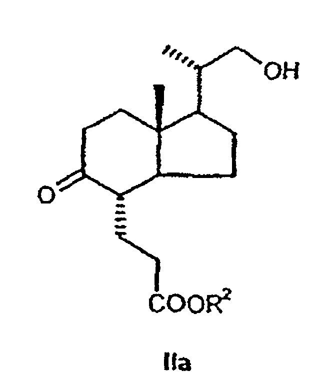 whose free hydroxy group is protected, to obtain a compound of general formula IIIa 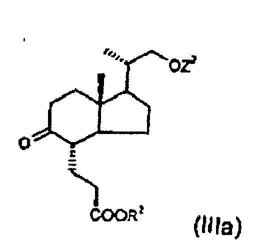 in which Z² has the meaning of Z, but does not mean hydrogen, and R² has the meaning of R¹, but does not mean hydrogen,
whose keto group is converted under common conditions into the tosylhydrazone Va 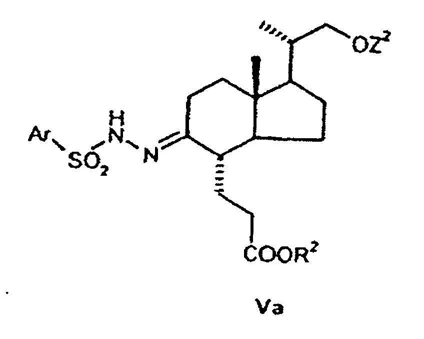 in which Ar means a phenyl group, which optionally is substituted in 1-3 places by a C₁-C₅-alkyl group,
which is reduced after reduction with a reducing agent such as sodium borohydride, sodium triacetoxy borohydride, lithium aluminium hydride, diisobutyl aluminium hydride or under Birch conditions to a compound of general formula VIa 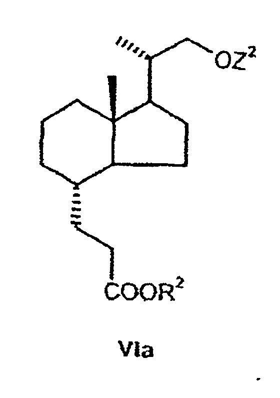 where if the protective groups are to have been cleaved under the conditions of reduction and their working-up, protective groups again are introduced, and their propionic acid side chain then is degraded by the reaction sequence
a) Introduction of a 2,3-double bond with lithium diisopropyl amide/DMPH/phenylselenyl bromide/hydrogen peroxide
b) Ozonolysis
c) Urea/hydrogen peroxide oxidation
d) Pyridinium chlorochromate oxidation and is converted into ketone Xa 26

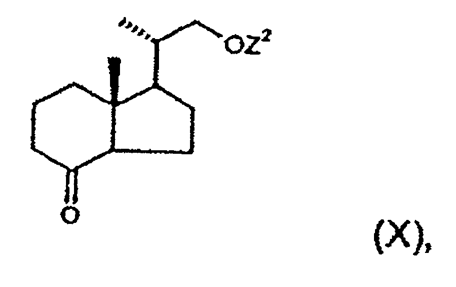

10. Intermediate products of the process according to Claim 9 of general formulae Va 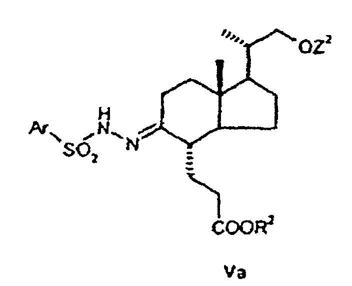 VIa. 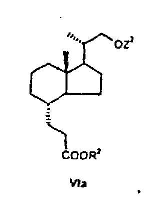 VIIa 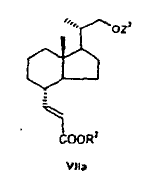 VIIIa 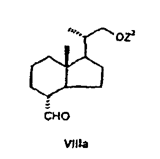 IXa 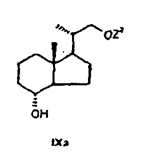 and Xa 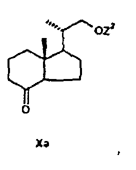 as well as their analogues, in which Z² has the meaning of Z and R² has the meaning of R¹, with the proviso that compounds of formula IXa in which Z² means tert-butyldimethylsilyl or hydrogen are excluded.

11. Use of the compounds of general formulae I, III, VIIa, and VIIIa for synthesis of vitamin D derivatives.

## Revendications

1. Composés de formule générale III 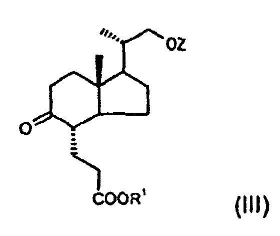 dans laquelle
R¹ signifie un atome d'hydrogène, un radical alkyle en C₁ à C₁₀, un radical alcényle en C₂ à C₁₀, un radical alcynyle en C₂ à C₁₀, les radicaux alkyle, alcényle et alcynyle pouvant être substitués par 1 à 5 atomes d'halogène, 1 à 5 groupes alcoxy en C₁ à C₅ et/ou être interrompus par 1 à 3 atomes d'oxygène ou 1 à 3 atomes de soufre, un radical perfluoroalkyle en C₁ à C₅, un radical perchloroalkyle en C₁ à C₅ ou un radical benzyle, qui est le cas échéant substitué par 1 à 2 atomes d'halogène, radicaux alkyle en C₁ à C₄, radicaux alcoxy en C₁ à C₄ et/ou groupes NO₂, et
Z signifie un atome d'hydrogène ou un groupe de protection d'une fonction hydroxy.

2. Composés de formule générale IV 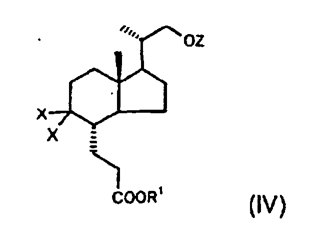 dans laquelle
R¹ signifie un atome d'hydrogène, un radical alkyle en C₁ à C₁₀, un radical alcényle en C₂ à C₁₀, un radical alcynyle en C₂ à C₁₀, les radicaux alkyle, alcényle et alcynyle pouvant être substitués par 1 à 5 atomes d'halogène, 1 à 5 groupes alcoxy en C₁ à C₅ et/ou être interrompus par 1 à 3 atomes d'oxygène ou 1 à 3 atomes de soufre, un radical perfluoroalkyle en C₁ à C₅, un radical perchloroalkyle en C₁ à C₅ ou un radical benzyle, qui est le cas échéant substitué par 1 à 2 atomes d'halogène, radicaux alkyle en C₁ à C₄, radicaux alcoxy en C₁ à C₄ et/ou groupes NO₂,
Z signifie un atome d'hydrogène ou un groupe de protection d'une fonction hydroxy et
X,X signifient ensemble un atome d'oxygène céto ou un groupe de protection de la fonction céto.

3. Composés de formule générale IV selon la revendication 2 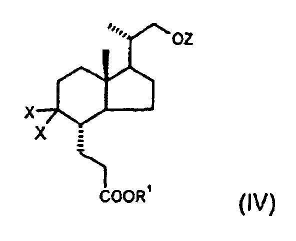 dans laquelle
R¹ signifie un atome d'hydrogène, un radical alkyle en C₁ à C₁₀, un radical alcényle en C₂ à C₁₀, un radical alcynyle en C₂ à C₁₀, les radicaux alkyle, alcényle et alcynyle pouvant être substitués par 1 à 5 atomes d'halogène, 1 à 5 groupes alcoxy en C₁ à C₅ et/ou être interrompus par 1 à 3 atomes d'oxygène ou 1 à 3 atomes de soufre, un radical perfluoroalkyle en C₁ à C₅, un radical perchloroalkyle en C₁ à C₅ ou un radical benzyle, qui est le cas échéant substitué par 1 à 2 atomes d'halogène, radicaux alkyle en C₁ à C₄, radicaux alcoxy en C₁ à C₄ et/ou groupes NO₂
Z signifie un atome d'hydrogène, un groupe (alkyle en C₁ à C₄) ₃Si, un groupe (C₆H₅) ₃Si, un groupe (alkyle en C₁ à C₅)₂(C₆H₅)Si, un groupe (alkyle en C₁ à C₅)(C₆H₅)₂Si, un groupe tétrahydrofurannyle, un groupe tétrahydropyrannyle, un groupe (C₁ à C₅)O(C₁ à C₅), un groupe benzyle le cas échéant substitué par 1 ou 2 atomes d'halogène, radicaux alkyle en C₁ à C₄, radicaux alcoxy en C₁ à C₄ ou groupes NO₂, un groupe -CO-(C₁ à C₆), ou un groupe benzoyle le cas échéant substitué par 1 ou 2 atomes d'halogène, radicaux alkyle en C₁ à C₄, radicaux alcoxy en C₁ à C₄ et/ou groupes NO₂, et
X,X signifie un groupe -O(C₁ à C₅), un groupe -S(C₁ à C₅), ensemble un atome d'oxygène céto, un groupe cyclique -O(C₂ à C₅) O-, ou un groupe cyclique -S(C₂ à C₅) S- ou un groupe C₆H₅-SO₂-NH-N, dont le radical phényle est le cas échéant monosubstitué à trisubstitué par un radical alkyle en C₁ à C₅.

4. Composé de formule I, selon la revendication 1

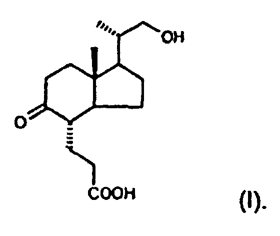

5. Procédé pour la préparation du composé de formule I selon la revendication 4, **caractérisé en ce que** le composé de formule I est obtenu par une réaction de dégradation du précurseur stéroïdien de formule 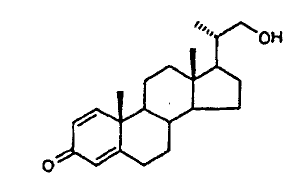 avec un microorganisme des genres Nocardioides ou Rhodococcus.

6. Procédé selon la revendication 5, **caractérisé en ce qu'**un des deux microorganismes est le Nocardioides simplex, ATCC 13260 ou le Rhodococcus sp. ATCC 13259.

7. Procédé pour le traitement ultérieur du composé de formule générale I 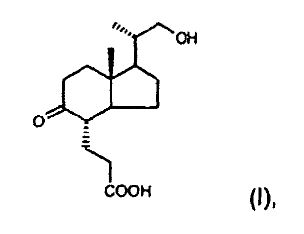 qui a été obtenu dans une étape microbiologique par dégradation du précurseur stéroïdien selon la revendication 5, **caractérisé en ce que** ses groupes fonctionnels sont, si souhaité, protégés dans un ordre quelconque, le groupe céto est le cas échéant transformé en un précurseur approprié, réduit en groupe méthylène et la chaîne acide propionique est dégradée dans un ordre de réaction quelconque en fonction céto et le cas échéant le groupe de protection de la fonction hydroxy est ensuite éliminé et/ou le cas échéant d'autres groupes de protection sont introduits et un composé de formule générale X 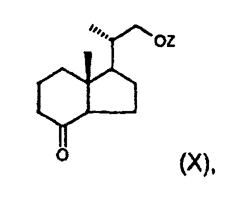 est obtenu, Z ayant la signification susmentionnée.

8. Procédé selon la revendication 7, **caractérisé en ce que** le composé de formule (X) est transformé davantage dans des étapes de réaction quelconques en vitamine D ou dérivés de la vitamine D.

9. Procédé selon la revendication 7, **caractérisé en ce que** le composé de formule I est transformé en un ester de formule générale IIa 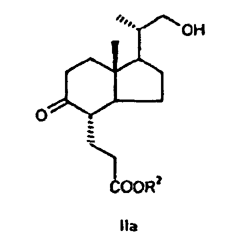 dont le groupe hydroxy libre est protégé pour obtenir un composé de formule générale IIIa, 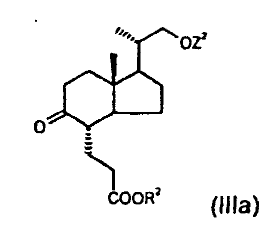 dans laquelle Z² a la signification de Z, mais ne signifie toutefois pas hydrogène et R² a la signification de R¹, mais ne signifie toutefois pas hydrogène, dont le groupe céto est transformé dans des conditions usuelles en tosylhydrazone Va 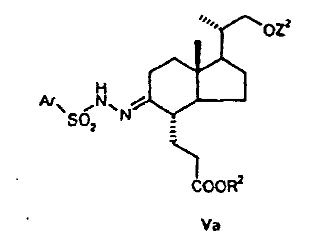 dans laquelle Ar signifie un groupe phényle qui est le cas échéant monosubstitué à trisubstitué par un groupe alkyle en C₁ à C₅,
qui est réduit, après réduction avec un réducteur tel que le borohydrure de sodium, le triacétoxyborohydrure de sodium, l'hydrure de lithium et d'aluminium, l'hydrure de diisobutylaluminium ou dans des conditions de Birch, en un composé de formule générale VIa 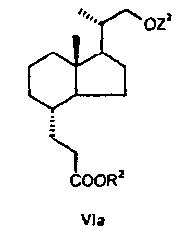 où, dans le cas où les groupes de protection seraient dissociés dans les conditions de réduction et son traitement, les groupes de protection sont réintroduits et
dont la chaîne latérale d'acide propionique est ensuite dégradée par une succession de réactions
a) introduction d'une double liaison 2,3 avec du diisopropylamide de lithium / DMPH / bromure de phénylsélényle / peroxyde d'hydrogène
b) ozonolyse
c) oxydation par urée/peroxyde d'hydrogène
d) oxydation par chlorochromate de pyridinium et transformée en cétone Xa

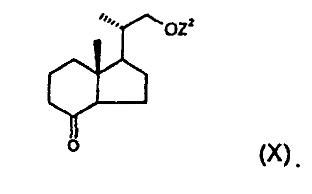

10. Produits intermédiaires du procédé selon la revendication 9 de formules générales
Va, 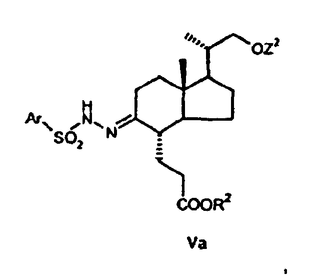 VIa, 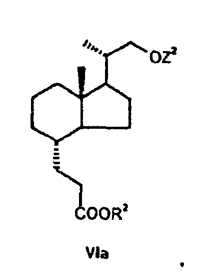 VIIa 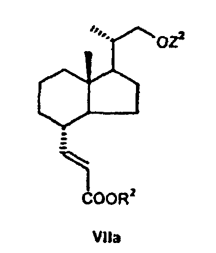 VIIIa 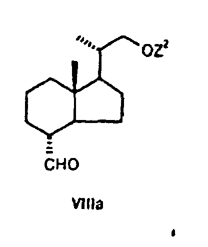 IXa 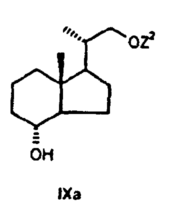 et
Xa 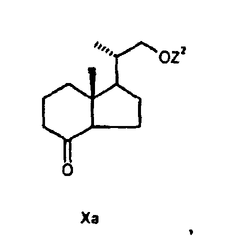 ainsi que leurs analogues, dans lesquels Z² a la signification de Z et R² la signification de R¹, à condition que les composés de formule IXa dans laquelle Z² signifie tert-butyldiméthylsilyle ou hydrogène soient exclus.

11. Utilisation des composés de formules générales I, III, VIIa et VIIIa pour la synthèse de dérivés de la vitamine D.
